# EUROPEAN PATENT APPLICATION

(11) **EP 4 640 834 A1**
(43) Date of publication of application: **29.10.2025**
(21) Application number: 23906979.2
(22) Date of filing: 18.12.2023
(51) Int. Cl.: C12N 15/53, A01H 1/00, A01H 5/00, C12N 5/10, C12N 9/02, C12N 15/63, C12P 7/22, C12P 7/26

(54) **FLAVANONE 2-HYDROXYLASE GENE AND USE OF SAME**

(30) Priority: 23.12.2022 JP 2022206702
(71) Applicant: Suntory Holdings Limited, Osaka-shi, Osaka 530-8203 (JP)
(72) Inventor: NAKAMURA, Noriko, Soraku-gun, Kyoto 619-0284 (JP); TANAKA, Yoshikazu, Soraku-gun, Kyoto 619-0284 (JP); AKASHI, Tomoyoshi, Fujisawa-shi, Kanagawa 252-0880 (JP); UCHIDA, Kai, Fujisawa-shi, Kanagawa 252-0880 (JP)
(74) Representative: Vossius & Partner Patentanwälte Rechtsanwälte mbB
(86) International application number: PCT/JP2023/045307
(87) International publication number: WO 2024/135619

(57) **Abstract**

Provided is a transgenic plant that has improved stress resistance and/or modified flower color, and modification to a blue flower color, or its inbred or outbred progeny, or their propagules, partial plant bodies, tissue or cells, as well as a method which allows easy and efficient production of 2-hydroxyflavanone. There is used a polynucleotide encoding an enzyme that belongs to the 2-oxoglutaric acid-dependent dioxygenase (ODD) family and that has hydroxylating activity at the 2-position of flavanones.

## Description

### FIELD

The present invention relates to novel polynucleotides coding for enzymes that belong to the 2-oxoglutaric acid-dependent dioxygenase (ODD) family and that have hydroxylating activity at the 2-position of flavanones, as well as to their use.

### BACKGROUND

Flavonoids, as typical plant secondary metabolites, have phenylalanine as the starting substance, and they are classified into numerous groups such as flavanones, flavones, isoflavone flavonols and anthocyanins, depending on the structure of the C-ring. The enzymes involved in their biosynthesis, and genes coding for the enzymes, have been well researched. Isoflavones and flavones are both biosynthesized from flavanones. For an isoflavone, isoflavone synthase catalyzes hydroxylation at position 2 of a flavanone and transfer of the B-ring (NPL 1), and then 2-hydroxyisoflavone dehydratase further acts to produce an isoflavone (NPL 2). Isoflavone synthase belongs to the cytochrome P450 family, while 2-hydroxyisoflavone dehydratase belongs to the carboxy esterase family. Flavones are present in numerous plants as aglycones, C-glycosides and O-glycosides (NPL 3). For example, plants of the family Caryophyllaceae, genus *Dianthus*, such as carnation (*Dianthus caryophyllus*) and nadeshiko (*Dianthus* sp.) produce flavone O-glycosides and flavone C-glycosides (NPL 4, NPL 5 and NPL 6). Flavone C-glycosides are also produced by gentian, horseradish, buckwheat and Poaceae plants. The functions of flavones in plants include protection from ultraviolet rays, interaction with microorganisms, protection from biological stress by production of phytoalexins, and blueing of anthocyanins by copigment action. For example, flavone C-glycosides such as isovitexin function as copigments for irises and gene recombinant carnations, producing blue flower color (NPL 7 and NPL 8).

Flavones are synthesized from flavanones by two routes (NPL 3). Flavone synthases catalyze the reaction for conversion of flavanones to flavones. For flavone synthases, genes coding for flavone synthase I belonging to the dioxygenase family (NPL 9) and flavone synthase II belonging to the cytochrome P450 family (NPL 10) are known. The produced flavones are metabolized into flavone O-glycosides or flavone C-glycosides by UDP-glucose-dependent glycosyltransferase. For example, the flavone O-glycosyltransferase gene of nemophila (NPL 11) and the flavone C-glycosyltransferase gene (NPL 12, NPL 13) of gentian and horseradish have already been isolated. Another route is via 2-hydroxyflavanone as an intermediate, where 2-hydroxyflavanone is produced first by the catalytic action of flavanone 2-hydroxylase (NPL 14). A 2-hydroxyflavanone C-glycoside is then produced by the action of glycosyltransferase at the 2-position hydroxyl group. This glycosyltransferase gene has already been reported (NPL 15). The 2-hydroxyflavanone C-glycoside is converted to a mixture of flavone 6-C-glycosides and flavone 8-C-glycosides (typically isovitexin and vitexin) by spontaneous dehydration reaction *in vitro* (NPL 15, NPL 16).

Enzymes associated with flavone biosynthesis differ in their molecular species depending on the plant species, and are thought to be related to enzymes belonging to the P450 family or enzymes belonging to the soluble 2-oxoglutaric acid-dependent dioxygenase (ODD) family, but it has not yet been known that enzymes belonging to the ODD family exhibit activity of specifically hydroxylating the 2-position of flavanones.

### [CITATION LIST]

### [NON PATENT LITERATURE]

[NPL 1] Plant Physiol. 1999 121:821-828
[NPL 2] Plant Physiol 2005 137:882-91
[NPL 3] Phytochemistry 2005 66:2399-2407
[NPL 4] Z. Naturforsch. 63c, 161D168(2008)
[NPL 5] Chem. Pharm. Bull. 59(9)1141-1148(2011)
[NPL 6] Phytochemistry. 2003 May; 63(1):15-23
[NPL 7] Plant Physiol Biochem. 2013 72:116-24
[NPL 8] Phytochemistry. 2003; 63:15-23
[NPL 9] Phytochemistry 2001 58:43-6
[NPL 10] Plant Cell Physiol. 1999 40:1182-6
[NPL 11] Plant Cell Physiol. 2018 59:2075-2085
[NPL 12] FEBS Lett. 2015 589:182-7
[NPL 13] Plant Cell Physiol. 2019 Dec 1; 60(12):2733-2743
[NPL 14] FEBS Lett. 1998 Jul 17; 431(2):287-90
[NPL 15] J Biol Chem. 2009 3; 284:17926-34
[NPL 16] Metabolic Engineering 2013 16:11-20

### SUMMARY

### [TECHNICAL PROBLEM]

It is an object of the present invention to provide a transgenic plant that has improved stress resistance and/or modified flower color, preferably modification to a blue flower color, or its inbred or outbred progeny, or their propagules, partial plant bodies, tissue or cells, as well as a method for easily and efficiently producing 2-hydroxyflavanone.

### [SOLUTION TO PROBLEM]

In most plant secondary metabolic systems, enzymes that carry out the same reaction have highly conserved nucleotide and amino acid sequences, even among different plant species. Specifically, these are homologous genes produced from common ancestral genes throughout branching speciation, and they are considered to be in orthologous relationships. However, the enzymes involved in the biosynthesis mechanism of flavones differ depending on the plant species, and it is conjectured that the metabolic systems have developed independently for each plant. As a result of much diligent research conducted using published RNA-sequence information and biochemical assay, the present inventors have found, surprisingly, that enzymes belonging to the ODD family derived from plants of the Caryophyllaceae family have activity of specifically hydroxylating the 2-position of flavanones, and the invention has been completed upon this discovery.

Specifically, the present invention provides the following.
[1] A polynucleotide encoding an enzyme that belongs to the 2-oxoglutaric acid-dependent dioxygenase (ODD) family and that has hydroxylating activity at the 2-position of flavanones, wherein the polynucleotide is selected from the group consisting of the following (A) to (E):
   (A) polynucleotides consisting of the nucleotide sequences of SEQ ID NO: 1, 3, 5, 7, 9 or 11;
   (B) polynucleotides that hybridize with polynucleotides consisting of nucleotide sequences complementary to the nucleotide sequences of SEQ ID NO: 1, 3, 5, 7, 9 or 11 under stringent conditions;
   (C) polynucleotides encoding proteins consisting of the amino acid sequences of SEQ ID NO: 2, 4, 6, 8, 10 or 12;
   (D) polynucleotides encoding proteins consisting of the amino acid sequences of SEQ ID NO: 2, 4, 6, 8, 10 or 12 having a deletion, substitution, insertion and/or addition of one or more amino acids; and
   (E) polynucleotides encoding proteins having amino acid sequences with at least 90% identity to the amino acid sequences of SEQ ID NO: 2, 4, 6, 8, 10 or 12.
[2] A polynucleotide according to [1], which is a polynucleotide consisting of the nucleotide sequence of SEQ ID NO: 1, 3, 5, 7, 9 or 11.
[3] A polynucleotide according to [1], which is a polynucleotide encoding a protein consisting of the amino acid sequence of SEQ ID NO: 2, 4, 6, 8, 10 or 12.
[4] A protein encoded by a polynucleotide according to any one of [1] to [3].
[5] A vector comprising a polynucleotide according to any one of [1] to [3].
[6] A transgenic plant, or its inbred or outbred progeny, that comprises a polynucleotide according to any one of [1] to [3].
[7] Propagules, partial plant bodies, tissue or cells of a transgenic plant according to [6] or its inbred or outbred progeny.
[8] Cut flowers of a transgenic plant according to [6], or its inbred or outbred progeny, or a processed form created from the cut flowers.
[9] A method for creating a transgenic plant, the method comprising a step of introducing into plant cells a polynucleotide encoding an enzyme that belongs to the 2-oxoglutaric acid-dependent dioxygenase (ODD) family and that has hydroxylating activity at the 2-position of flavanones.
[10] The method according to [9], wherein the polynucleotide is selected from the group consisting of the following (A) to (E):
   (A) polynucleotides consisting of the nucleotide sequences of SEQ ID NO: 1, 3, 5, 7, 9 or 11;
   (B) polynucleotides that hybridize with polynucleotides consisting of nucleotide sequences complementary to the nucleotide sequences of SEQ ID NO: 1, 3, 5, 7, 9 or 11 under stringent conditions;
   (C) polynucleotides encoding proteins consisting of the amino acid sequences of SEQ ID NO: 2, 4, 6, 8, 10 or 12;
   (D) polynucleotides encoding proteins consisting of the amino acid sequences of SEQ ID NO: 2, 4, 6, 8, 10 or 12 having a deletion, substitution, insertion and/or addition of one or more amino acids; and
   (E) polynucleotides encoding proteins having amino acid sequences with at least 90% identity to the amino acid sequences of SEQ ID NO: 2, 4, 6, 8, 10 or 12.
[11] A non-human host containing a polynucleotide according to any one of [1] to [3].
[12] A method for producing an enzyme with hydroxylating activity at the 2-position of flavanones, the method comprising:
   a step of culturing a non-human host according to [11], and
   a step of harvesting from the non-human host an enzyme with hydroxylating activity at the 2-position of flavanones.
[13] A method for producing 2-hydroxyflavanone,
   wherein the method includes contacting an enzyme produced by the method according to [12] with a flavanone.

### [ADVANTAGEOUS EFFECTS OF INVENTION]

According to the invention it is possible to provide a transgenic plant that has improved stress resistance and/or modified flower color, and modification to a blue flower color, or its inbred or outbred progeny, or their propagules, partial plant bodies, tissue or cells. According to the invention it is also possible to provide a non-human host (for example, yeast or bacteria) that expresses an enzyme with hydroxylating activity at the 2-position of flavanones, and to use it to easily and efficiently produce 2-hydroxyflavanone.

### BRIEF DESCRIPTION OF DRAWINGS

Fig. 1 shows the biosynthetic pathway for flavone C-glycoside in plants, via 2-hydroxyflavanone as an intermediate.
Fig. 2 shows high-performance liquid chromatograms of a crude protein solution extract from *E. coli* expressing SvF2H-1 or SvF2H-2, and of an enzyme reaction solution obtained by enzyme reaction on naringenin.
Fig. 3 shows high-performance liquid chromatograms of a crude protein solution extract from *E. coli* expressing AgF2H or DsF2H-1, and of an enzyme reaction solution obtained by enzyme reaction on naringenin.
Fig. 4 shows high-performance liquid chromatograms of a crude protein solution extract from yeast expressing D_DN4746 or Dca16994.1, and of an enzyme reaction solution obtained by enzyme reaction on naringenin.

Plant flowers and leaves contain flavones as pigments. Flavones are organic compounds that are flavan-derived cyclic ketones, and in plants they mainly exist as glycosides. Flavone, in the strict definition, refers to 2,3-didehydroflavan-4-one, which is a compound with chemical formula C₁₅H₁₀O₂ and molecular weight 222.24, but in the wider sense flavones are a category of flavonoids, a flavonoid being classified as a "flavone" if it has a flavone structure as the basic backbone and also lacks the hydroxyl group at the 3-position.

In plants, flavones are also distributed as glycosides, in addition to the free form, such that flavone O-glycosides and flavone C-glycosides are mainly produced, but flavone C-glycosides in particular are known to exhibit powerful copigment action, with blue color being exhibited by intermolecular interaction in the co-presence of anthocyanins (a pigment group abundantly found in plants), and especially delphinidin-type anthocyanins such as delphinidin, malvidin and petunidin. Copigment action includes not only a color depth effect that induces blue color production, but also a deep color effect or an effect of increasing color stability.

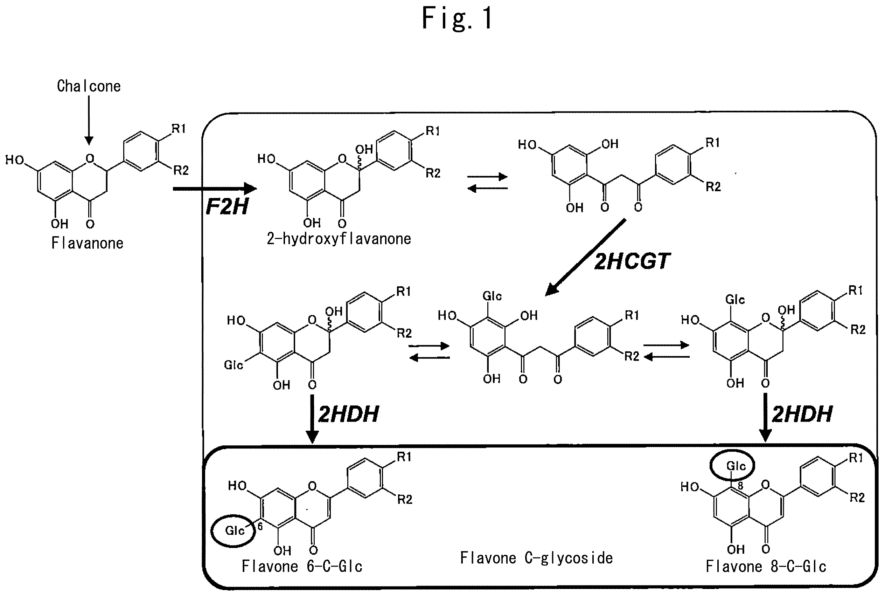

As used herein, "flavone C-glycoside" means a glycoside of a flavone in the wide sense, i.e. a derivative falling under the definition of flavones, wherein an aglycone is directly bonded to the anomeric carbon of an aldose. Flavone C-glycosides include, but are not limited to, luteolin C-glycoside, tricetin C-glycoside, apigenin C-glycoside and acacetin C-glycoside. Flavone C-glycosides also include glycosides of apigenin, luteolin, tricetin and acacetin derivatives. Two routes are known for the biosynthetic pathway of flavone C-glycosides in plants. Of these, flavone 6-C-glucoside and flavone 8-C-glucoside are produced by the action of flavanone 2-hydroxylase (F2H), flavone C-glycosylase (2HCGT) and dehydratase (2HDH) in a pathway via 2-hydroxyflavanone as the intermediate (Fig. 1). It is therefore conjectured that by incorporating the flavanone 2-hydroxylase (F2H) gene as one of the essential genes of this pathway into a host plant, it would be possible to accumulate flavone C-glycoside in the plant cells to modify the plant flower color.

The present inventors have succeeded in obtaining a novel polynucleotide coding for an enzyme that belongs to the 2-oxoglutaric acid-dependent dioxygenase (ODD) family and that has hydroxylating activity at the 2-position of flavanones. The present invention relates to a polynucleotide encoding an enzyme that belongs to the 2-oxoglutaric acid-dependent dioxygenase (ODD) family and that has hydroxylating activity at the 2-position of flavanones.

The enzyme family 2-oxoglutaric acid-dependent dioxygenase (ODD) is a water-soluble dioxygenase containing divalent iron, and it catalyzes a variety of oxidation reactions such as hydroxylation and demethylation of various biomolecules ranging from low molecular compounds to proteins and DNA. ODD is widely abundant among bacteria, plants and animals, and although approximately 60 2OGD genes are present in humans, and 2OGD genes make up 0.5% of many plant genomes, no classifying nomenclature method has yet been established based on evolutionary lineage analysis.

Enzymes belonging to the ODD family to be used for the invention will typically be derived from Caryophyllaceae plants, examples of Caryophyllaceae plants including, but not being limited to, bladder campion (*Silene vulgaris*), fringed pink (*Dianthus superbus*), corncockle (*Agrostemma githago*), carnation (*Dianthus caryophyllus*) and nadeshiko (*Dianthus hybrida*).

The polynucleotide of the invention is preferably selected from the group consisting of the following (A) to (E):
(A) polynucleotides consisting of the nucleotide sequences of SEQ ID NO: 1, 3, 5, 7, 9 or 11;
(B) polynucleotides that hybridize with polynucleotides consisting of nucleotide sequences complementary to the nucleotide sequences of SEQ ID NO: 1, 3, 5, 7, 9 or 11 under stringent conditions;
(C) polynucleotides encoding proteins consisting of the amino acid sequences of SEQ ID NO: 2, 4, 6, 8, 10 or 12;
(D) polynucleotides encoding proteins consisting of the amino acid sequences of SEQ ID NO: 2, 4, 6, 8, 10 or 12 having a deletion, substitution, insertion and/or addition of one or more amino acids; and
(E) polynucleotides encoding proteins having amino acid sequences with at least 90% identity to the amino acid sequences of SEQ ID NO: 2, 4, 6, 8, 10 or 12.

SEQ ID NO: 1 is the nucleotide sequence of a gene coding for a protein (SvF2H-1) having hydroxylating activity at the 2-position of flavanone derived from bladder campion, SEQ ID NO: 3 is the nucleotide sequence of a gene coding for a protein (SvF2H-2) having hydroxylating activity at the 2-position of flavanone derived from bladder campion, SEQ ID NO: 5 is the nucleotide sequence of a gene coding for a protein (DsF2H-1) having hydroxylating activity at the 2-position of flavanone derived from fringed pink, SEQ ID NO: 7 is the nucleotide sequence of a gene coding for a protein (AgF2H) having hydroxylating activity at the 2-position of flavanone derived from corncockle, SEQ ID NO: 9 is the nucleotide sequence of a gene coding for a protein (D_DN4746) having hydroxylating activity at the 2-position of flavanone derived from nadeshiko, and SEQ ID NO: 11 is the nucleotide sequence of a gene coding for a protein (Dca16994.1) having hydroxylating activity at the 2-position of flavanone derived from carnation.

The term "polynucleotide" as used herein refers to DNA or RNA.

Also as used herein, the term "stringent conditions" refers to conditions that allow specific binding between a polynucleotide or oligonucleotide and genomic DNA in a selective and detectable manner. Stringent conditions are defined by an appropriate combination of salt concentration, organic solvent (for example, formamide), temperature and other known conditions. Specifically, stringency is increased by reducing the salt concentration, increasing the organic solvent concentration or raising the hybridization temperature. Stringency is also affected by the rinsing conditions after hybridization. The rinsing conditions are defined by the salt concentration and temperature, and stringency of rinsing is increased by reducing the salt concentration and raising the temperature. Therefore, the term "stringent conditions" means conditions such that specific hybridization takes place only between nucleotide sequences with high identity, such as a degree of "identity" between the nucleotide sequences of about 80% or greater, preferably about 90% or greater, more preferably about 95% or greater, even more preferably 97% or greater and most preferably 98% or greater, on average. The "stringent conditions" may be, for example, a temperature of 60°C to 68°C, a sodium concentration of 150 to 900 mM and preferably 600 to 900 mM, and a pH of 6 to 8, with specific examples including hybridization under conditions of 5 × SSC (750 mM NaCl, 75 mM trisodium citrate), 1% SDS, 5 × Denhardt solution, 50% formaldehyde, 42°C, and rinsing under conditions of 0.1 × SSC (15 mM NaCl, 1.5 mM trisodium citrate), 0.1% SDS, 55°C.

The hybridization may be carried out by a method that is publicly known in the field or a similar method, such as the method described in Current Protocols in Molecular Biology (edited by Frederick M. Ausubel et al., 1987). When a commercially available library is to be used, the hybridization may be carried out according to the method described in the accompanying directions for use. The gene selected by hybridization may be naturally derived, such as plant-derived or non-plant-derived. The gene selected by the hybridization may be cDNA, genomic DNA or chemically synthesized DNA.

SEQ ID NO: 2 is the amino acid sequence of a protein (SvF2H-1) having hydroxylating activity at the 2-position of flavanone derived from bladder campion, SEQ ID NO: 4 is the amino acid sequence of a protein (SvF2H-2) having hydroxylating activity at the 2-position of flavanone derived from bladder campion, SEQ ID NO: 6 is the amino acid sequence of a protein (DsF2H-1) having hydroxylating activity at the 2-position of flavanone derived from fringed pink, SEQ ID NO: 8 is the amino acid sequence of a protein (AgF2H) having hydroxylating activity at the 2-position of flavanone derived from corncockle, SEQ ID NO: 10 is the amino acid sequence of a protein (D_DN4746) having hydroxylating activity at the 2-position of flavanone derived from nadeshiko, and SEQ ID NO: 12 is the amino acid sequence of a protein (Dca16994.1) having hydroxylating activity at the 2-position of flavanone derived from carnation.

The phrase "amino acid sequence having a deletion, substitution, insertion and/or addition of one or more amino acids" means an amino acid sequence having a deletion, substitution, insertion and/or addition of 1 to 20, preferably 1 to 5 and more preferably 1 to 3 arbitrary amino acids. Site-directed mutagenesis is a useful genetic engineering method as it allows introduction of specific mutations into specified sites, and it may be carried out by the method described in Molecular Cloning: A Laboratory Manual, 2nd Ed., Cold Spring Harbor Laboratory Press, Cold Spring Harbor, N.Y., 1989. By expressing the mutant DNA using a suitable expression system, it is possible to obtain a protein consisting of an amino acid sequence having a deletion, substitution, insertion and/or addition of one or more amino acids. A polynucleotide can be obtained by a method that is publicly known to those skilled in the art, such as a method of chemical synthesis using the phosphoramidite method, or a nucleic acid amplification method using a plant nucleic acid specimen as template, and primers designed based on the nucleotide sequence of the target gene.

Throughout the present specification, the term "identity" means, for polypeptide sequences (or amino acid sequences) or polynucleotide sequences (or nucleotide sequences), the quantity (number) of amino acid residues or nucleotides composing them that can be determined to be identical between the two chains, in the sense of mutual agreement between them, meaning the degree of sequence correlation between two polypeptide sequences or two polynucleotide sequences, and this "identity" can be easily calculated. Numerous methods are known for measuring identity between two polynucleotide sequences or polypeptide sequences, and the term "identity" is well known to those skilled in the art (for example, see Lesk, A.M. (Ed.), Computational Molecular Biology, Oxford University Press, New York, (1988); Smith, D.W. (Ed.), Biocomputing: Informatics and Genome Projects, Academic Press, New York, (1993); Grifin, A.M. & Grifin, H.G. (Ed.), Computer Analysis of Sequence Data: Part I, Human Press, New Jersey, (1994); von Heinje, G., Sequence Analysis in Molecular Biology, Academic Press, New York (1987); Gribskov, M. & Devereux, J. (Ed.), Sequence Analysis Primer, M-Stockton Press, New York, (1991) and elsewhere).

Also, the numerical values for "identity" used in the present specification, unless otherwise specified, may be the numerical values calculated using an identity search program known to those skilled in the art, but they are preferably numerical values calculated using the ClustalW program of MacVector Application (version 9.5, Oxford Molecular Ltd., Oxford, England). According to the invention, the degree of "identity" between amino acid sequences is, for example, about 90% or greater, preferably about 95% or greater, more preferably about 97% or greater, even more preferably about 98% or greater and most preferably about 99% or greater.

The polynucleotide (nucleic acid, gene) of the invention "encodes" a protein of interest. The term "encodes" also includes both encoding a structural sequence (exon) that is a continuous section of the protein of interest, and encoding via an intervening sequence (intron). Also, the term "encodes" includes both encoding a structural sequence (exon) that is a continuous section of the protein of interest, and encoding via an intervening sequence (intron).

A gene with a natural nucleotide sequence can be obtained by analysis using a DNA sequencer, for example. DNA encoding an enzyme having a modified amino acid sequence can be synthesized using common site-directed mutagenesis or PCR, based on DNA having the natural nucleotide sequence. For example, a DNA fragment to be modified may be obtained by restriction enzyme treatment of natural cDNA or genomic DNA, and used as template for site-diriected mutagenesis or PCR using primers with the desired mutation, to obtain a DNA fragment having the desired modification. The DNA fragment having the mutation may then be linked with a DNA fragment encoding another portion of the target enzyme.

Alternatively, in order to obtain DNA encoding an enzyme consisting of a shortened amino acid sequence, it is possible to cut DNA encoding an amino acid sequence longer than the target amino acid sequence, such as the full-length amino acid sequence, with a desired restriction enzyme, and if the obtained DNA fragment does not code for the full target amino acid sequence, then a DNA fragment consisting of the sequence of the missing portion may be synthesized and linked to it.

By expressing the obtained polynucleotide using a gene expression system in *E. coli* or yeast and measuring the enzyme activity, it is possible to confirm that the obtained polynucleotide encodes a protein with the desired activity.

The present invention relates to a (recombinant) vector, and especially an expression vector, comprising the aforementioned polynucleotide. The vector of the invention also comprises an expression control region, such as a promoter, terminator and replication origin, that are dependent on the type of host plant into which it is introduced. Examples of promoters that constitutively express polynucleotides in plant cells include cauliflower mosaic virus 35S promoter, El₂35S promoter having two 35S promoter enhancer regions linked together, and the rd29A gene promoter, rbcS promoter and mac-1 promoter. For tissue-specific gene expression, a promoter for a gene expressed specifically in that tissue may be used.

The vector may be created by a common method using a restriction enzyme and ligase. Transformation of a host plant using the expression vector may also be carried out by a common method.

The invention further relates to a transgenic plant containing the aforementioned polynucleotide or vector, or to its inbred or outbred progeny.

At the current level of technology for creation of transgenic plants, it is possible to use techniques to introduce a polynucleotide into a plant and constitutively or tissue-specifically express the polynucleotide. Transfer of the DNA into the plant may be carried out by a method known to those skilled in the art, such as the *Agrobacterium* method, binary vector method, electroporation method, PEG method or particle gun method.

Plants to be used as hosts for the invention are not particularly restricted and may be plants belonging to the genus Rosaceae *Rosa,* Solanaceae *Petunia,* Compositae *Chrysanthemum,* Caryophyllaceae *Dianthus* or Liliaceae *Lilium,* among which rose cultivar of Rosaceae *Rosa* (scientific name: *Rosa hybrida*) is especially preferred. The term "rose plant", as used herein, is a rose cultivar of Rosaceae *Rosa* (scientific name: *Rosa hybrida*), which is its taxonomical classification. Roses are largely classified as Hybrid Tea, Floribunda and Polyantha roses based on their tree form and flower size, with the major pigment (anthocyanin) in the petals of all lines being of two types, the cyanidin-type and pelargonidin-type. The type of rose plant used as a host for the invention is not particularly restricted, and any of these varieties or lines are suitable. Examples of rose varieties to be used as hosts include Ocean Song, Noblesse, Rita Perfumera, Cool Water, Fame, Topless and Peach Avalanche.

By introducing a polynucleotide of the invention into plant cells, the flavone C-glycoside accumulates in the plant cells and acts as phytoalexin, thereby improving the stress resistance and exhibiting copigment action in the co-presence of delphinidin-type anthocyanins, and allowing creation of a transgenic plant with a flower color modified to blue flower color, or its inbred or outbred progeny.

The invention still further relates to cut flowers of the obtained transgenic plant or its inbred or outbred progeny, or the propagules, partial plant body, tissue or cells, or a processed form created from the cut flowers (especially processed cut flowers). The processed cut flowers referred to here include pressed flowers formed using cut flowers, or preserved flowers, dry flowers or resin sealed products, with no limitation to these.

The invention also relates to a non-human host comprising the aforementioned polynucleotide or vector. The non-human host used may be a prokaryote or eukaryote. A prokaryote may be a common host, such as a bacterium belonging to the genus *Escherichia*, such as *Escherichia coli*, or a *Bacillus* microorganism such as *Bacillus subtilis.* Eukaryotes used may be lower eukaryotic organisms such as eukaryotic microorganisms, examples of which are fungi such as yeasts or filamentous fungi as fungi. Examples of yeasts include *Saccharomyces* microorganisms such as *Saccharomyces cerevisiae*, and as filamentous fungi, *Aspergillus* microorganisms such as *Aspergillus oryzae*, *Aspergillus niger* and *Penicillium* microorganisms. A non-human host used may also be animal cells or plant cells, where animal cells used may be cell lines from mice, hamsters, monkeys or humans, and insect cells, such as silkworm cells or the silkworms themselves, may also be used as the host.

The invention still further relates to a method for producing an enzyme with hydroxylating activity at the 2-position of flavanones, the method comprising a step of incorporating a polynucleotide of the invention into a non-human host, a step of culturing the non-human host, and a step of harvesting from the non-human host an enzyme with hydroxylating activity at the 2-position of flavanones, and a method of producing 2-hydroxyflavanone, as well as to a method of contacting a flavanone with an enzyme produced by the method. This method allows easy and efficient production of 2-hydroxyflavanone.

The present invention will now be explained in greater detail by examples.

### EXAMPLES

### [Example 1: Cloning of full-length cDNA of candidate genes coding for proteins having hydroxylating activity at 2-position of flavanone derived from bladder campion, and functional analysis]

After acquiring transcriptome data for bladder campion (SRA037583) from the SRA database of DDBJ, contigs were constructed by *de novo* assembly using Trinity. The constructed contigs were used for TBLASTN analysis using the amino acid sequence of *Arabidopsis thaliana* F3H (AT3G51240) as query, and two candidates (SvF2H-1 and SvF2H-2) with approximately 60% identity were selected.

The nucleotide sequence of the SvF2H-1 gene is as follows.

ATGACAAAAGAGGAATCAATAACCGTGCCCGAATGGGAAGGCGAGAAATCCGTGA
AGCAAACTTACGTTAGGGACGAAGATGACCGTCCTAAGGTCGCTTACAACGAATTC
AGCGACGACATTCCCGTCATTTCCCTCTCTGGTATCGACTCAGCCGACGAGAGGGCT
CACATTCGCAGCCAAATCACTGAGGCCTGCGAGAAATACGGCATTTTCCAGGTCGTA
AACCACGGTATTGACACTGACCTCATCTCTACCATGGCTCGTCTTTGTTCCGAGTTTT

TTAATTTGCCGGGTCATGAGAAGCAACGTTTTGGTGCTTCTGAAGGTAAACCCGGGA
GCTTCTTCGTCTCTAGCCCCTTCAAGGGTGAGTTGGTAAAAAATTGGAGAGAAACGG
TGACGCAATGCACATTCCCAATCAAGAGCGGGGACTACACACTATGGCCCGACAAG
CCCGACGGATGGAAAGCCGTGACTAAGGAATACAGCGAAAAAATGATGGACCTTTC
CCACCAGCTGCTTGGGCTACTATCAGAGGCCATGGGTCTTGAGACAGAGGCCCTAA
GCAAGGCCTGTGTTACAATGGACCAAAGGGTTATGATTAACTACTACCCAAAATGC
CCTCAGCCAGACCTCACCCTTGGCCTTCCCCGGCACACCGACCCCGGGACCATCACC
CTTCTCTACCAGGACCTGGTTGGTGGGCTACAAGCCACTCGCGATGATGGTAAGACT
TGGATCACTGTCCCGCCGATTGCTGGTGCTCTTGTTGTCAACCTTGGTGATCATGCTT
ATTACGTAAGTAATGGAAGGTTTACAAGTGCGGAACACCGGGCCGTGGTGAACTCA
AACCAGAGTAGGTTGTCAATAGCAACATTCCAATACCCAACAGCTGAGGCAACGGT
CTTCCCATTGGCGATAAGGCAAGGAGAGAAGCCGGTAATGGAAGAAGCTATCACCT
TCGCGGAGATGTATAGAAGGAAGATGAGCTCGGACATTGAGCGTCCGAAGCTTAAG
AAGCTTGATGGTAAAATGGACAAGTCCAAGTTAGAGTCTAGGCCCATCGGCGAGAT
CTTTGTTTGA (SEQ ID NO: 1)

The amino acid sequence of SvF2H-1 is as follows.

MTKEESITVPEWEGEKSVKQTYVRDEDDRPKVAYNEFSDDIPVISLSGIDSADERAHIRS
QITEACEKYGIFQVVNHGIDTDLISTMARLCSEFFNLPGHEKQRFGASEGKPGSFFVSSPF
KGELVKNWRETVTQCTFPIKSGDYTLWPDKPDGWKAVTKEYSEKMMDLSHQLLGLLS
EAMGLETEALSKACVTMDQRVMINYYPKCPQPDLTLGLPRHTDPGTITLLYQDLVGGL
QATRDDGKTWITVPPIAGALVVNLGDHAYYVSNGRFTSAEHRAVVNSNQSRLSIATFQY
PTAEATVFPLAIRQGEKPVMEEAITFAEMYRRKMSSDIERPKLKKLDGKMDKSKLESRPI
GEIFV*(SEQ ID NO: 2)

The nucleotide sequence of the SvF2H-2 gene is as follows.

ATGACAAAAGAAAAGGCAACAACGTTGACCGAGCTCCAAGACCAAAACTGCATCA
ACCAAAGTTTCGTAAGAGACGAGGATGACCGTCCTAAGGTCGCCTACAACGACTTT
ACCGACGTTATCCCCGTCATTTCACTCTCCGGCATTGACGCCCACGATGAAAGGGCT
GAAATTCGTCGTAAGATCACTGAAGCTTGCGAAACTTGGGGCCTTTTCCAGGTCATC
GACCATGGTGTTGAACTTGAAATTATATCGGAGATGGCACGCCTTTCTACCGAGTTT
TTTCACTTGCCCGCGTCTGAGAAGCAGCAGTTTGCTGCTTCCGATGGCAAGCCGGGA
AGTTTCTTTGTTTCAAGCCCTTTTAAGAGAGAGTTGGTACAGAACTGGAGAGAGACA
GTAACACAATGCACATACCCAATTAAGAGTGGGGACTACACATTATGGCCAGATAA
ACCTGAAGGATGGCGAAAAATAACCAAAGAATACAGTGATAAAATGATGGAGCTA
AGTCACAAACTCCTTGGGATTCTTTCTGAGGCAATGGGTTTAGAGACGGACGCGTTA

AGCAAGGCTTGTGTCGAAATGGATCAAAGGGTTATGATCAATTATTACCCTAAGTGC
CCTCAACCTGACCTCACCCTTGGGCTCCTTCGCCACACCGATCCTGGTACCATTACTC
TTCTTTACCAGGATCANGTTGGTGGGCTTCNNGCCACTTGTGATGATGGTGTGACTT
GGNTTACGGTTCCTCCTATTCCTGGCGCGCTTGTTGTTAACCTTGGTGATCATTCCCA
TTTTGTGAGCAATGGAAGGTTCGTGAGTGCGGAGCACAGGGCAGTGGTGAACTCGA
ACACAGGCAGGCTGTCGATGGTGACATTTCAGTATCCGACAGCAGAGGCGACAGTG
TACCCCTTGACGCTGAGAGAAGGGGAGACGCCGATCATGGAAGAGGCCATAACCTT
CGGGGAGATGTACAGGAGGAAGATGAGCAAAGACCTTGAGCTTCCTCGGAACAAG
AAGCTAGACACTAAGATGGAAAAGTCGGAATTAGAGTCAAGACCCATTGACGCCAT
CTTTATTTAA (SEQ ID NO: 3)

The amino acid sequence of SvF2H-2 is as follows.

MTKEKATTLTELQDQNCINQSFVRDEDDRPKVAYNDFTDVIPVISLSGIDAHDERAEIRR
KITEACETWGLFQVIDHGVELEIISEMARLSTEFFHLPASEKQQFAASDGKPGSFFVSSPF
KRELVQNWRETVTQCTYPIKSGDYTLWPDKPEGWRKITKEYSDKMMELSHKLLGILSE
AMGLETDALSKACVEMDQRVMINYYPKCPQPDLTLGLLRHTDPGTITLLYQDXVGGLX
ATCDDGVTWXTVPPIPGALVVNLGDHSHFVSNGRFVSAEHRAVVNSNTGRLSMVTFQY
PTAEATVYPLTLREGETPIMEEAITFGEMYRRKMSKDLELPRNKKLDTKMEKSELESRPI
DAIFI*(SEQ ID NO: 4)

Seeds of bladder campion (Campion Bladder, Mikasa Gardening) were placed on filter paper wetted with sterilized water, and germinated for 6 days under conditions of brightness (16 hours)/darkness (8 hours). The plant seedlings were recovered and the total RNA was isolated using an SV Total RNA Isolation System (Promega), after which a SuperScriptIII Reverse Transcriptase (Invitrogen) was used for synthesis of cDNA. The cDNA was used as template for amplification of CDS using primers (SvF2H: 5'-ATGACAAAAGAGGAATCAATAACCG-3' (SEQ ID NO: 13) and 5'-TCAAACAAAGATCTCGCCGATGG-3' (SEQ ID NO: 14); SvF2H2: 5'-ATGACAAAAGAAAAGGCAACAACG-3' (SEQ ID NO: 15), and 5'-TTAAATAAAGATGGCGTCAATGGG-3' (SEQ ID NO: 16)), and using PrimeSTAR MAX DNA Polymerase (Takara). The amplification product was introduced into pCR8/GW/TOPO Vector (Invitrogen) after addition of dA using TaKaRa Ex Taq (Takara). In addition, LR reaction using Gateway LR Clonase II Enzyme Mix (Invitrogen) was carried out to transfer pET-53-DEST vector (Novagen), for transformation of *E. coli* Rosetta2 (DE3). The control vector used was one having the GUS gene introduced into pET-53-DEST vector.

The *E*. *coli* was cultured overnight at 37°C with 10 mL of LB liquid medium (50 mg/L kanamycin), and then 10 mL of the preculturing solution was added to 240 mL of the same LB liquid medium and the mixture was shake cultured at 37°C to an OD600 of 0.4 to 0.6. Next, IPTG was added to a final concentration of 0.5 mM and culturing was continued for 4 hours at 28°C. After collecting the cells by centrifugal separation (9,000 x g, 2 min), they were disrupted with glass beads in 50 mM Tris-HCl (pH 8.0). The supernatant obtained by centrifugal separation (4°C, 9,000 x g, 10 minutes) was used in an enzyme assay. The substrate S-naringenin (10 µg) and crude enzyme solution were added in the presence of 50 mM Tris-HCl (pH 8.0), 1 mM oxoglutaric acid, 0.4 mM FeSO₄ and 4 mM Na ascorbate, and the mixture was incubated for 10 minutes at 30°C. The reaction mixture was distributed with ethyl acetate and the ethyl acetate layer was analyzed by HPLC.

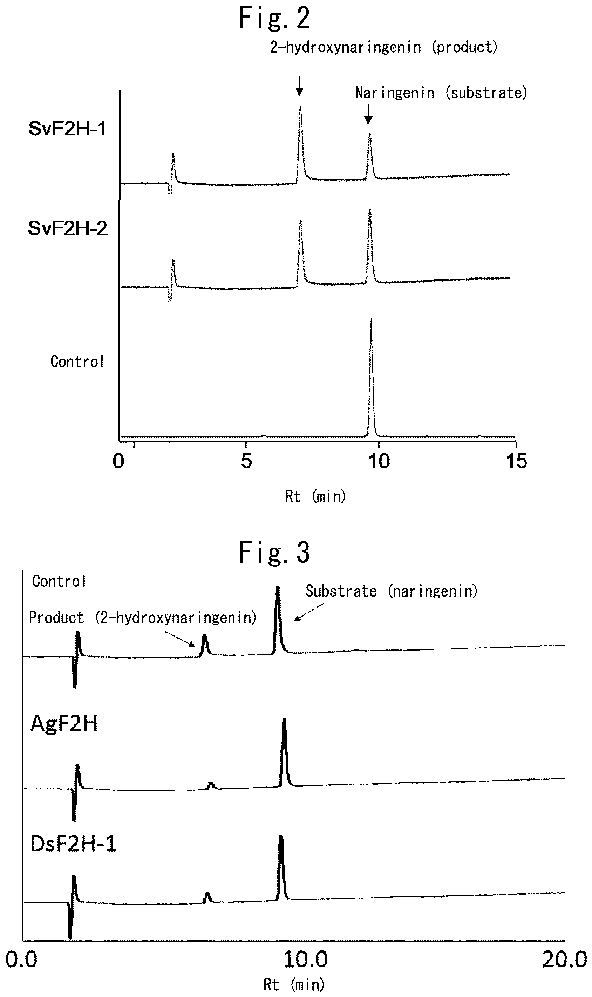

This demonstrated that in reaction with SvF2H-1 and SvF2H-2, 2-hydroxynaringenin is produced from S-naringenin (Fig. 2).

### [Example 2: Isolation of candidate genes coding for proteins having hydroxylating activity at the 2-position of flavanone derived from corncockle and fringed pink, and their functional analysis]

### <Selection of candidate genes>

An F2H search was conducted for corncockle and fringed pink. Contigs were constructed by *de novo* assembly based on published RNA-sequencing information. A search was conducted for sequences exhibiting identity of 75% or greater with SvF2H as estimated amino acids, and three candidates (DsF2H-1, DsF2H-2, AgF2H) were selected.

The nucleotide sequence of the DsF2H-1 gene is as follows.

ATGACAAAAGAAGATAAAAAAAATGTTACTACATTGACAGAATTAGTAGACCAAAA
ATGCATTAACCAAAGCTTCGTAAGAGACGAAGACGAGCGTCCGAAAGTCGCTTACA
ACGAATTTAGCGACGAAATTCCCGTCATTTCGCTACTTGGCATTGATGATGACAGCG
AGAGGACTCACATTCGTCGTAAGATTACCGAGGCGTGTGAGTCTTGGGGTATTTTTC
AGGTCGTTGACCATGGAGTTGACCTCTTACTCATTTCGGAAATGGCTCGGCTTAGTA
CCGAGTTTTTTAACTTGCCCGCTCATGAGAAGCAGCGGTTCGATACCTCGGGCGGTA
AGCCCGGGAGTTTTTTCGTGTCGAGTCCTTTTAAGGGGGAACTAGTTCAGAACTGGC
GAGAGACGGTCACACAATGCACGTACCCGATCAAGAGCGGAGACTTCAACCTATGG
CCGGATAAGCCAGAGGGTTGGAGAAAAGTAACCAAAGACTACGGTGACAAAATGA
TGGGCCTAAGTCACAAGATACTTGGGCTTTTATCTGAAGCTATGGGTCTCGAGACCG
AGGCCCTTAGCAAGGCCTGTGTTGAGATGGACCAAAGGGTTATGATCAACTACTAC
CCTAAGTGCCCTCAACCCGACTTAACCCTTGGCCTTCTCCGCCACACTGATCCTGGT
ACAATTACTCTTTTGTACCAAGATCAAGTTGGCGGTCTCCAAGCCACCCGTGATGAC

GGTGTGACTTGGATCACTGTTACGCCTGTTCCTGGTGCCCTTGTCGTCAATCTTGGTG
ATCATGCCTATTACGCGAGTAACGGAAGGTTCACAAGTGCGGAACACCGAGCAGTG
GTGAACTCGAATCAAGGCAGGCTGTCGATGGCGACATTCCAATACCCGACAGCGGA
GGCAATGGTGTATCCGTTGACAGTCAGTGAAGGCGAGAAGCCGGTGATGGAAGAGG
CCATCACCTTCGCGGAAATGTACCGTAGGAAAATGAGCAAGGACCTTGAGCGTCCT
CACCTTAAGAAGCTCGACAATACGATGGACAAGTCAAAATTAGAGGGTCGGGCCAT
TAATGAGATCTTTATCTAA (SEQ ID NO: 5)

The amino acid sequence of DsF2H-1 is as follows.

MTKEDKKNVTTLTELVDQKCINQSFVRDEDERPKVAYNEFSDEIPVISLLGIDDDSERTHI
RRKITEACESWGIFQVVDHGVDLLLISEMARLSTEFFNLPAHEKQRFDTSGGKPGSFFVS
SPFKGELVQNWRETVTQCTYPIKSGDFNLWPDKPEGWRKVTKDYGDKMMGLSHKILG
LLSEAMGLETEALSKACVEMDQRVMINYYPKCPQPDLTLGLLRHTDPGTITLLYQDQV
GGLQATRDDGVTWITVTPVPGALVVNLGDHAYYASNGRFTSAEHRAVVNSNQGRLSM
ATFQYPTAEAMVYPLTVSEGEKPVMEEAITFAEMYRRKMSKDLERPHLKKLDNTMDKS
KLEGRAINEIFI*(SEQ ID NO: 6)

The nucleotide sequence of the AgF2H gene is as follows.

ATGCCTCAAGAAGAGTCAATAATAACCGTGAGTGAGATGCAAGAGGATAAATCCGT
GGAGCAAAAATTCGTTAGGGACGAAGATGACCGTCCTAAGGTCGCATACAACGAAT
TTAGCGACGACATTCCCGTGATTTCCCTCTCCGGCATTGACTCCCCCGACGACAGGG
ACCACATTCGCCGCCAAGTCACAGAGGCCTGCGAGGAATACGGCATTTTCCAAGTG
GTAGACCATGGTATCGACTCTGATATCATCTCGACCATGGCTCGTCAATCTTCCCAG
TTTTTCAACTTGCCCGCTCCTGAAAAGCAGCGTTTCGCTGCTGCCGAGGGTAAACCT
GGCAGCTTCTTCGTCTCTAGTCCCTTCAAGGGTGAGTTGGTGCAAAATTGGAGAGAA
ACGGTGACGCAGTGCACGTTCCCGATCAAGAGTGGAGACTACACGCTGTGGCCGGA
CAAGCCGGAGGGATGGAGAAGCGTGACCAAGGAGTACAGTGAAAAAATGATGGAT
CTTTCTCACAAACTCCTCGGGGTGCTGTCCGAGGCAATGGGTCTGGAGACGGAGGCC
CTAAGCAATGCCTGCGTTACAATGGACCAACGGGTTATGATTAATTACTATCCCAAG
TGCCCTCAACCGGACCTCACCCTCGGTCTCCCCCGCCACACCGACCCTGGCACCATC
ACCCTTCTCTACCAGGACCTCGTTGGTGGCCTCCAAGCCACTCGCGATGATGGTAAG
ACTTGGATTACTGTCCCGCCTATTCCCGGTGCTCTTGTCGTCAACCTTGGCGATCATG
CCTTTTACGCAAGCAACGGAAGGTTCACAAGTGCGGAACACCGGGCAGTGGTGAAC
TCAAACCAGAGCAGGTTGTCAATAGCGACATTCCAATATCCGACAGCGGAGGCAAT
GGTGTACCCCTTAGCAATAAGGGAAGGAGAGACAGCGGTGATGGAAGAGGCCATC
ACGTTTGCGGAGATGTATAGGAGGAAGATGAGCTCCGACATTGAGCGACCTAAGCA

TAAGAAGGTCGATGGTAAAATGGACAAGTCCAAGTTAGAGTCTAGGCCCATGGAAG
AGATCTTTGTTTGA (SEQ ID NO: 7)

The amino acid sequence of AgF2H is as follows.

MPQEESIITVSEMQEDKSVEQKFVRDEDDRPKVAYNEFSDDIPVISLSGIDSPDDRDHIRR
QVTEACEEYGIFQVVDHGIDSDIISTMARQSSQFFNLPAPEKQRFAAAEGKPGSFFVSSPF
KGELVQNWRETVTQCTFPIKSGDYTLWPDKPEGWRSVTKEYSEKMMDLSHKLLGVLSE
AMGLETEALSNACVTMDQRVMINYYPKCPQPDLTLGLPRHTDPGTITLLYQDLVGGLQ
ATRDDGKTWITVPPIPGALVVNLGDHAFYASNGRFTSAEHRAVVNSNQSRLSIATFQYP
TAEAMVYPLAIREGETAVMEEAITFAEMYRRKMSSDIERPKHKKVDGKMDKSKLESRP
MEEIFV*(SEQ ID NO: 8)

### <cDNA synthesis>

The total RNA was extracted and purified from corncockle (approximately 40 mg) and fringed pink (40 mg) within 7 days of germination, using ISOSPIN Plant RNA (Nippon Gene Co., Ltd.). The concentration of the purified total RNA was measured using Nano drop, and the purity was confirmed by electrophoresis with 1% agarose gel. Next, SuperScript IV Reverse Transcriptase (Invitrogen) was used for reverse transcription reaction with each purified total RNA and oligo dT primer, to synthesize cDNA.

### <Cloning and transformation of candidate genes>

Each cDNA synthesized above was used for PCR to amplify the candidate gene sequence together with the heat-resistant polymerase Prime STAR MAX (Takara Bio, Inc.). The reaction system, the primers used and the PCR conditions were as follows.

### (Reaction system)

| | |
|---|---|
| 2 × Prime STAR MAX Premix | 20 µl |
| Forward primer | 1 µl |
| Reverse primer | 1 µl |
| Template (cDNA) | 1 µl |
| Water (DNAse RNAse Free) | 17 µl |

### (Primers used)

### DsF2H-1

Forward: ATGACAAAAGAAGATAAAAAAAATGTTAC (SEQ ID NO: 17)
Reverse: TTAGATAAAGATCTCATTAATGGCC (SEQ ID NO: 18) DsF2H-2
Forward: ATGACAAAGGAAACACCCTCGACCG (SEQ ID NO: 19)
Reverse: CTACACGAAGATTTCTCCAATAGC (SEQ ID NO: 20)

### AgF2H

Forward: ATGCCTCAAGAAGAGTCAATAATAAC (SEQ ID NO: 21)
Reverse: TCAAACAAAGATCTCTTCCATGGGC (SEQ ID NO: 22)

### (PCR conditions)

98°C, 1 min → (98°C, 5 sec → 53°C, 15 sec → 72°C, 1 min) × 35 cycles → 72°C, 1 min → 12°C

### <Purification of target DNA>

The amplified candidate genes were treated for purification of the target DNA using a Wizard SV Gel and PCR Clean-Up System (Promega), and their concentrations were measured with Nano drop.

### <Addition of A to 3'-end of target DNA>

After mixing 10 µl of target DNA and 10 µl of 2 × SapphireAmp Fast PCR Master Mix (Takara Bio, Inc.), A was added to the 3'-end of the target DNA with a thermal cycler, under conditions of 60°C, 15 min.

### <pCR8/GW/TOPO and annealing>

The following reaction system was reacted under conditions of room temperature, 15 min, to transfer the A-added product into the pCR8/GW/TOPO vector (Invitrogen) (T vector) of the transfer vector.

### (Reaction system)

| | |
|---|---|
| A-added product | 1 µl |
| Salt solution: | 1µl |
| pCR8/GW/TOPO vector | 1 µl |
| Water (DNase RNase Free) | 3 µl |

### <Transformation>

After placing 3 µl of the gene-transferred T vector in 100 µl of *E. coli* DH5α that had been melted on ice, it was slowly mixed and cooled for 5 minutes in a refrigerator. Next, 100 µl of S.O.C. Medium (Invitrogen) was added to minimal medium for *E. coli,* and shake culturing was carried out for 30 minutes under conditions of 37°C, 75 min⁻¹ without stirring. Next, the total amount of bacteria solution was added to the spectinomycin (Spe)-added LB agar medium [LB Medium (Miller), granulated (Kanto Kagaku Co., Ltd.), 25 g/L; AGAR, 15 g/L; 0.1 mM screening antibiotic], a spreader was used to spread out the bacteria solution, and culturing was carried out at 37°C for 16 to 20 hours.

### <Recovery of target plasmids and next-generation sequencing>

The screened colonies were added to 2 ml of Spe-containing LB liquid medium [LB Medium, granulated, 25 g/L, 0.1 mM screening antibiotic], and a rotating incubator was used for culturing overnight at 37°C. A Wizard Plus SV Minipreps DNA purification System (Promega) was used for recovery of the cultured plasmids, and the concentration was measured with Nano drop. Next-generation sequencing was carried out to confirm that no mutation had occurred in the plasmids. PCR and ethanol precipitation were carried out to prepare sequences. The reaction system conditions for PCR were as follows.

### (Reaction system)

| | |
|---|---|
| Big Dye | 1 µl |
| ABI buffer | 3.5 µl |
| GW1 or GW2 Primer | 2 µl |

(Dilution with 50 µM primer:water = 2:48)

| | |
|---|---|
| Plasmid | X µl |

(300 to 500 ng, matched to plasmid size)
Water (DNAse RNAse Free) 13.5-X µl

### (PCR conditions)

96°C, 1 min → (96°C, 10 sec → 50°C, 5 sec → 60°C, 4 min) × 30 cycles → 4°C

For ethanol precipitation, the total amount of the sequencing reaction product was transferred to a 1.5 ml tube, and then 5 µl of 0.125 M EDTA and 60 µl of 99.5% ethanol were added and the mixture was mixed with inversion several times and then allowed to stand at room temperature for 15 minutes. The mixture was then centrifuged at 4°C, 12,000 rpm for 15 minutes, the solution was discarded with a Pipetman while avoiding disruption of the pellets, 100 µl of 70% ethanol was added, and the mixture was centrifuged at 4°C, 12,000 rpm for 10 minutes. The solution was again discarded with a Pipetman while avoiding disruption of the pellets, and after drying for 3 to 5 minutes with an aspirator, it was submitted to the General Research Institute of Nihon University, College of Bioresource Sciences, with a request for DNA sequence analysis.

### <Introduction of target plasmid expression vector>

The colonies used were selected based on the data obtained from the sequence results, for introduction into the pET-53-DEST vector. The reaction system used was a thermal cycler under conditions of 25°C, 30 minutes, as follows.

### (Reaction system)

| | |
|---|---|
| Entry clone (plasmid prepared in 2.9) | 1 µl |
| Destination vector (pET-53-DEST: 75 ng/µl) | 1 µl |
| Water (DNAse RNAse Free) | 2 µl |
| LR ClonaseII Enzyme Mix (Invitrogen) | 1 µl |

Next, 1 µl of Proteinase K (Invitrogen) was added to the reaction system, and the thermal cycler was used under conditions of 37°C, 10 minutes. After the reaction, 3 µl of the reaction mixture was added to 100 µl of DH5α, and the mixture was slowly mixed and allowed to stand for 5 minutes in a refrigerator. Preincubated carbenicillin (Car)-added LB agar medium was smeared and culturing was conducted overnight at 37°C.

### <Recovery of target plasmids and next-generation sequencing>

The grown colonies were added to 2 ml of Car-containing LB liquid medium, and a rotating incubator was used for culturing overnight at 37°C. A Wizard Plus SV Minipreps DNA Purification System was used for recovery of the cultured plasmids, and the concentration was measured with Nano drop. A next-generation sequencer was used to confirm that no mutation had occurred in the plasmids. PCR and ethanol precipitation were carried out to prepare sequences. The reaction system conditions for PCR were as follows.

### (Reaction system)

| | |
|---|---|
| Big Dye | 1 µl |
| ABI buffer | 3.5 µl |
| pET Upstream Primer | 2 µl |

(Dilution with 50 µM primer:water = 2:48)

| | |
|---|---|
| Plasmid | X µl |

(300 to 500 ng, matched to plasmid size)
Water (DNAse RNAse Free) 13.5-X µl

### (PCR conditions)

96°C, 1 min → (96°C, 10 sec → 50°C, 5 sec → 60°C, 4 min) × 30 cycles → 4°C

For ethanol precipitation, the total amount of the sequencing reaction product was transferred to a 1.5 ml tube, and then 5 µl of 0.125 M EDTA and 60 µl of 99.5% ethanol were added and the mixture was mixed with inversion several times and then allowed to stand at room temperature for 15 minutes. The mixture was then centrifuged at 4°C, 12,000 rpm for 15 minutes, the solution was discarded with a Pipetman while avoiding disruption of the pellets, 100 µl of 70% ethanol was added, and the mixture was centrifuged at 4°C, 12,000 rpm for 10 minutes. The solution was again discarded with a Pipetman while avoiding disruption of the pellets, and after drying for 3 to 5 minutes with an aspirator, a request was made for DNA sequence analysis.

### <Transformation of E. coli for expression>

After dissolving 50 µl of *E. coli* Rosetta2 (DE3) with respect to F2H on ice, 2 µl of plasmids of the colonies confirmed by sequence analysis to have the transferred plasmids was added and the mixture was smeared onto preincubated Car-containing LB agar medium at 37°C, and cultured overnight at 37°C.

### <F2H preculturing, main culturing and expression induction>

After adding one colony of transformed Rosetta2 (DE3) to 10 ml of Car-containing LB liquid medium, culturing was carried out overnight with a shaking incubator under conditions of 37°C, 180 rpm. On the following day, the total amount of preculturing solution was added to 240 ml of Car-containing LB liquid medium, and shake culturing was carried out at 37°C, 180 rpm until the OD600 reached 0.4 to 0.6. IPTG was then added to a final concentration of 0.5 mM for shake culturing.

Upon completion of the shake culturing, culturing was repeated under conditions of 4°C, 9000 x g, 2 min, and the cells alone were collected.

### <Preparation of E. coli disruptate with F2H>

The collected cells were placed on ice, cooled glass beads were added to the 5 ml level of the tube used, and Binding Buffer was added to the 10 ml level. A procedure of applying a vortex for 30 seconds and cooling on ice for 30 seconds was repeated a total of 3 times. After this, a procedure of centrifuging under conditions of 4°C, 9000 x g, 10 min, recovering the supernatant liquid alone and again adding Binding Buffer to the 10 ml level was repeated 3 times. The recovered supernatant liquid was filtered with a 0.45 µm filter.

The composition of Binding Buffer was as follows.

### Final concentration

| | |
|---|---|
| Potassium phosphate buffer (pH 7.5) | 20 mM |
| Imidazole | 40 mM |
| Sodium chloride | 500 mM |
| Distilled water | for adjustment |

### <Purification of enzyme proteins>

After flowing 10 ml of distilled water into a HisTrap HP (GE Healthcare) column to remove the inner ethanol, 10 ml of Binding Buffer was flowed in to equilibrate the column. The filtered supernatant liquid was flowed in and adsorbed onto the column, and then Binding Buffer was again passed through to 10 ml to wash the column. After washing, 5 ml of Elution Buffer was flowed into the column and fractionated into 10 Eppendorf tubes at 500 µl each. After collecting 5 µl from each fraction, the mixture was mixed with 100 µl of Protein Assay Bradford Reagent (Wako) by inverted stirring, and the blue-colored fraction was transferred onto ice.

A 25 ml portion of 0.1 M potassium phosphate buffer (pH 7.5) was flowed through a Hi-Trap Desalting (GE Healthcare) column, for washing and equilibration. The fraction that had been transferred onto ice was then passed through the column, and 10 ml of 0.1 M potassium phosphate buffer (pH 7.5) was flowed in and fractionated into 10 Eppendorf tubes at 500 µl each. After recovering 5 µl from each fraction, the mixture was mixed with 100 µl of Protein Assay Bradford Reagent by inverted stirring, the blue-colored fraction was recovered, the protein concentration was measured by the Bradford method, and SDS-PAGE was conducted for qualitative analysis. The SDS-PAGE conditions used were XL-Ladder as the molecular weight marker and SuperSep^{™} Ace 10-20 with 13 wells as a precast gel plate, and the electrophoresis conditions were 20 mA, with flow until the marker reached the bottom edge of the gel. The used HisTrap column was washed with 10 ml of distilled water, and 10 ml of 20% ethanol was flowed through prior to cold storage. The desalting column was washed with 25 ml of 20% ethanol prior to cold storage.

The composition of the Elution Buffer was as follows.

### Final concentration

| | |
|---|---|
| Potassium phosphate buffer (pH 7.5) | 20 mM |
| Imidazole | 500 mM |
| Sodium chloride | 500 mM |
| Distilled water | for adjustment |

### <Enzyme assay for F2H>

The S-form naringenin used as substrate was dissolved in methanol. After using 2-methoxyethanol to dissolve the determined amount of air-dried S-form naringenin and adding Tris-HCl (pH 8.0), oxoglutaric acid, FeSO₄, Na ascorbate and distilled water, enzyme was added and the mixture was incubated under conditions of 30°C, 70 min⁻¹. Ethyl acetate was subsequently added, and the mixture was subjected to a vortex and centrifuged under conditions of 4°C, 9000 x g, 2 minutes for distribution. After centrifugation, the ethyl acetate layer was recovered and air-dried, after which it was dissolved in 100 µl of methanol and analyzed by HPLC.

### (Reaction system)

### Final concentration

| | |
|---|---|
| 2-Methoxyethanol | 3/100 of total amount |
| Tris-HCl (pH 8.0) | 50 mM |
| Oxoglutaric acid | 1 mM |
| FeSO₄ | 0.4 mM |
| Na ascorbate | 4 mM |
| Distilled water | for adjustment |
| Enzyme solution | q.s |

**[Table 1-1]**

| Gene name | Reaction time | Substrate concentration range | Enzyme amount |
|---|---|---|---|
| SvF2H-1 | 15 min (30 min w/ 5 µM alone) | 5 µM - 100 µM | 2.5 µg |
| SvF2H-2 | 15 min | 5 µM - 50 µM | 1 µg |
| AgF2H | 15 min | 2.5 µM - 50 µM | 1 µg |
| DsF2H-1 | 15 min | 1 µM - 10 µM | 0.5 µg |

**[Table 1-2]**

| Gene name | Reaction system total | Added ethyl acetate amount | Recovered ethyl acetate amount |
|---|---|---|---|
| SvF2H-1 | 1 ml | 500 µl | 300 µl |
| SvF2H-2 | 3 ml | 1000 µl | 500 µl |
| AgF2H | 1 ml | 500 µl | 300 µl |
| DsF2H-1 | 1 ml | 500 µl | 300 µl |

Since protein expression could not be confirmed for DsF2H-2, no assay was carried out.

### <HPLC analysis>

The obtained assay product was analyzed under the following HPLC analysis conditions.

### (HPLC analysis conditions)

Column: TSK-Gel ODS-80TM
Flow rate: 1.0 ml/min
Column temperature: 40°C
Gradient: Solvent A 40% → 100% (20 min)
Solvent B 60% → 0% (20 min)
Solvent A: methanol (TFA 0.1%)
Solvent B: ultrapure water (TFA: 0.1%)
Detection wavelength: 280 nm

### <Results of functional analysis of AgF2H and DsF2H-1>

Upon assay of the purified AgF2H and DsF2H-1 with naringenin and verifying the results using HPLC, it was confirmed that 2-hydroxynaringenin had been produced in the same manner as the control SvF2H-1 (Fig. 3).

### [Example 3: Isolation of candidate genes coding for proteins having hydroxylating activity at 2-position of flavanones derived from nadeshiko and carnation, and their functional analysis]

### <Isolation of total RNA from nadeshiko>

Using an RNeasy Plant Mini Kit (QIAGEN), the total RNA was isolated from the petals and leaves of the commercially available nadeshiko product Miite Raspberry Rose (Suntory Flowers), according to the manufacturer's recommended protocol.

### <Expression analysis of nadeshiko-derived cDNA>

A library was prepared from the total RNA for provision to the next-generation sequencer NextSeq 500, using a SureSelect Strand-Specific RNA library preparation kit (Agilent Technologies) according to the manufacturer's recommended protocol. The prepared library was sequenced using a NextSeq 500 (Illumina Co.), and the obtained reads were analyzed. The reads from all of the samples were then combined and assembled using a Trinity v2.12.0, to determine the contig sequences. The obtained contig sequences were used for mapping of pair reads for each sample using an RSEM 1.3.0, and the FPKM value was calculated to determine the expression level.

### <Estimation of gene function>

The obtained contig sequences were used for a DIAMOND search in NCBI NR and a BLAST search (E-value ≤ 1.0) in Araport11, followed by function annotation (gene function estimation).

### <Isolation of full-length cDNA for candidate genes derived from nadeshiko and carnation>

A BLAST search was conducted using the amino acid sequence of SvF2H-1 from among the obtained contig sequences, selecting D_DN4746 (nadeshiko-derived) which had the highest degree of identity. A BLAST search was similarly conducted using the amino acid sequence of SvF2H-1 in the Carnation DB (http://carnation.kazusa.or.jp/), selecting Dca16994.1 (carnation-derived) which had the highest degree of identity. Full-length cDNA clones were obtained by artificial gene synthesis based on the assembled full-length cDNA sequence. The identity at the amino acid level between D_DN4746 and Dca16994.1 was 98.9%.

The nucleotide sequence of the D_DN4746 gene is as follows.

ATGACAAAGGAAACACCCTCGACCGTAACGGTATGGGAAAACGAGACATCCGTCAA
GCAAAAGTTTGTACGAGATGAGGACGACCGCCCTAAGGTCGCCTACAACGAATTCA
GCGATGAAATTCCTGTCATTTCGTTGAATGGTATTGACACCGACAATGAGAGGGCCC
ACATTCGTAGCAAGATCACCGAGGCTTGCGAGACGTGGGGCATCTTTCAAGTCGTG
GACCATGGGGTTGACCCTAATCTCGTATCTGAAATGGCTCGACTTTCTACCGAGTTT
TTCAATTTGCCGATTCACGAGAAGCTCCGCTTTGGTGCTTCTGACGGTAAGCCTGGC
AGCTTCTTTGTGTCGAGCCCCTTTAAGGGGGAGCTAGTGAAAAACTGGAGGGAGAC
GGTGACACAATGCACGTACCCAATCAAGAGCGGAGACTACACCCTATGGCCGGATT
CACCGGACGGTTGGAAAAATGTGACAAAAGAGTACAGTGAAAAAATGATGGACCT
AACACACAAGCTCCTTGGGGTTTTGTCCGAGGCTATGGGTCTGGAAACCGAAGCCTT
AAGCAAGGCGTGTGTCGATATGGACCAAAGGGTTATGATCAATTACTACCCTAAGT
GCCCGCAACCTGACCTCACCCTTGGTCTACCCCGCCACACTGATCCTGGTACCATCA
CGCTTTTGTACCAGGACCAGGTTGGCGGTCTCCAAGCTACTCGCGATGATGGCAAGT
CTTGGATTACTGTCCCGCCTGTTGCTGGTGCCCTTGTCGTCAACCTTGGTGATCACGC
CTACTATGTGAGCAACGGGAGGTTCACGAGTGCTGAACACAGAGCAGTGGTGAACT
CAAACCAGAGCAGGCTATCGATAGCGACATTCCAGTATCCGACAGCCGAGGCAACC
ATCTACCCATTAGCGATTCGAGAAGGCGAGGAACCTGTGATGGAAGAAGCCATCAC
TTTTGCGGAAATGTATAGGAGAAAGATGAGCTCGGATATCGAGCGTCCTAAGCTTA
AGAAGTTGGATGGTACAATGGACAAGTCCAAGTTAGAAGCTAGAGCTATTGGAGAA
ATCTTCGTGTAG (SEQ ID NO: 9)

The amino acid sequence of D_DN4746 is as follows. 
MTKETPSTVTVWENETSVKQKFVRDEDDRPKVAYNEFSDEIPVISLNGIDTDNERAHIRS
KITEACETWGIFQVVD
 
HGVDPNLVSEMARLSTEFFNLPIHEKLRFGASDGKPGSFFVSSPFKGELVKNWRETVTQ
CTYPIKSGDYTLWPDSP
 
DGWKNVTKEYSEKMMDLTHKLLGVLSEAMGLETEALSKACVDMDQRVMINYYPKCP
QPDLTLGLPRHTDPGTITLL
 
YQDQVGGLQATRDDGKSWITVPPVAGALVVNLGDHAYYVSNGRFTSAEHRAVVNSNQ
SRLSIATFQYPTAEATIYP
 LAIREGEEPVMEEAITFAEMYRRKMSSDIERPKLKKLDGTMDKSKLEARAIGEIFV*(SEQ ID NO: 10)

The nucleotide sequence of the Dca16994.1 gene is as follows.

ATGACAAAGGAAGCACCCTCGACCGTAACGGTATGGGAAAACGAGACATCCGTCAA
GCAAAAGTTTGTACGAGATGAGGACGACCGCCCTAAGGTCGCCTACAACGAATTCA
GCGATGAAATTCCTGTCATTTCGTTGAATGGTATTGACACAGACAATGAGAGAGCCC
ACATTCGTAGCAAGATCACCGAGGCTTGCGAGACGTGGGGCATCTTTCAAGTCGTG
GACCATGGGTTTGATCCTAATCTCGTATCTGAAATGGCTCGACTTTCTACCGAGTTTT
TCAATTTGCCGATTCACGAGAAGCTCCGCTTTGGTGCTTCTGACGGTAAGCCTGGCA
GCTTCTTTGTGTCGAGCCCCTTTAAGGGGGAGCTAGTGAAAAACTGGAGGGAGACG
GTGACACAATGCACGTACCCAATCAAGAGCGGAGACTACACCCTATGGCCGGATTC
ACCGGACGGTTGGAAAAATGTGACAAAAGAGTACAGTGAAAAAATGATGGACCTA
ACACACAAGCTCCTTGGGGTTTTGTCCGAGGCTATGGGTCTGGAAACTGAAGCCTTA
AGCAAGGCGTGTGTCGATATGGACCAAAGGGTTATGATCAATTACTACCCTAAGTG
CCCGCAACCTGACCTCACCCTTGGTCTACCCCGCCACACTGATCCTGGTACCATCAC
GCTTTTGTACCAGGACCAGGTTGGCGGTCTCCAAGCTACTCGCGATGATGGCAAGTC
TTGGATTACTGTCCCGCCTGTTGCTGGTGCCCTTGTCGTCAACCTTGGTGATCACGCC
TACTATGTGAGCAACGGGAGGTTCACGAGTGCTGAACACAGAGCAGTGGTGAACTC
AAACCAGAGCAGGCTATCTATAGCGACATTCCAGTATCCGACAGCCGAGGCAACCA
TCTACCCATTAGCGATTCGAGAAGGCGAGGAACCTGTGATGGAAGAAGCCATCACT
TTTGCGGAAATGTATAGGAGAAAGATGAGCTCGGATATCGAGCGTCCTAAGCAGAA
GTTGGATGGTACAATGGACAAGTCCAAGTTAGAAGCTAGAGCTATTGGAGAAATCT
TCGTGTAA (SEQ ID NO: 11)

The amino acid sequence of Dca16994.1 is as follows.

MTKEAPSTVTVWENETSVKQKFVRDEDDRPKVAYNEFSDEIPVISLNGIDTDNERAHIRS
KITEACETWGIFQVVDHGFDPNLVSEMARLSTEFFNLPIHEKLRFGASDGKPGSFFVSSPF
KGELVKNWRETVTQCTYPIKSGDYTLWPDSPDGWKNVTKEYSEKMMDLTHKLLGVLS
EAMGLETEALSKACVDMDQRVMINYYPKCPQPDLTLGLPRHTDPGTITLLYQDQVGGL
QATRDDGKSWITVPPVAGALVVNLGDHAYYVSNGRFTSAEHRAVVNSNQSRLSIATFQ
YPTAEATIYPLAIREGEEPVMEEAITFAEMYRRKMSSDIERPKQKLDGTMDKSKLEARAI
GEIFV*(SEQ ID NO: 12)

Upon examining the identity at the amino acid level between D_DN4746 and F2H genes of Caryophyllaceae plants belonging to the ODD family (SvF2H-1, SvF2H-2, AgF2H and DsF2H-1), the identity with SvF2H-1 was 87.5%, the identity with SvF2H-2 was 76.2%, the identity with AgF2H was 81.5% and the identity with DSF2H-1 was 81.3%. However, the identities at the amino acid level with F2H genes of plants belonging to P450 (rice (*Oryza sativa*)-derived flavanone 2-hydroxylase (OsF2H), licorice (*Glycyrrhiza echinata* L.)-derived flavanone 2-hydroxylase (GeF2H) and barrelclover (*Medicago truncatula*)-derived flavanone 2-hydroxylase (MtF2H)) were 7.7%, 7.8% and 8.1%, respectively. Upon examining the identity at the amino acid level between Dca16994.1 and F2H genes of Caryophyllaceae plants belonging to the ODD family (SvF2H-1, SvF2H-2, AgF2H and DsF2H-1), the identity with SvF2H-1 was 87%, the identity with SvF2H-2 was 75.6%, the identity with AgF2H was 81.2% and the identity with DSF2H-1 was 80.5%. However, the identities at the amino acid level with F2H genes of plants belonging to P450 (rice (*Oryza sativa*)-derived flavanone 2-hydroxylase (OsF2H), licorice (*Glycyrrhiza echinata* L.)-derived flavanone 2-hydroxylase (GeF2H) and barrelclover (*Medicago truncatula*)-derived flavanone 2-hydroxylase (MtF2H)) were 7.9%, 8.2% and 7.3%, respectively. Therefore, D_DN4746 and Dca16994.1 are clearly distinguished from F2H genes belonging to P450. This demonstrated that the genes of the invention are completely different from enzyme genes of the P450 family.

### <Construction of yeast expression vectors>

D_DN4746 and Dca16994.1, as candidate proteins which have hydroxylating activity at the 2-position of flavanones, were used to prepare yeast expression vectors pSPB8380 and pSPB8379 containing the full length of D_DN4746 or Dca16994.1, using pESC-TRP (Agilent Technologies) according to the manufacturer's recommended protocol.

### <Expression and enzyme reaction of flavanone 2-hydroxylase in yeast>

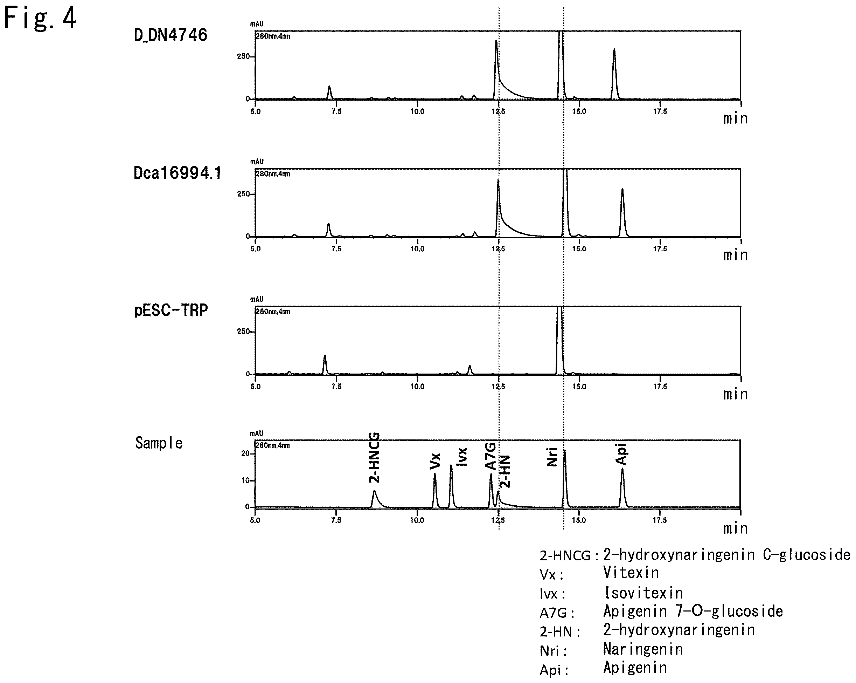

Plasmids pSPB8380 and pSPB8379 were introduced into yeast strain YPH499 (Agilent Technologies) by the manufacturer's recommended protocol, and transformed yeasts were obtained. Protein expression was induced in the transformed yeasts using pESC Yeast Epitope Tagging Vectors (Agilent Technologies) by the manufacturer's recommended protocol, and *in vivo* enzyme reaction was carried out by addition of 2 µg/mL hemin and 100 µM naringenin during culturing. The obtained culture solution was centrifuged (3000 rpm, 4°C, 10 minutes), and the supernatant was collected and then purified with Sep-Pak C18, 6 cc Vac Cartridge (500 mg) (Japan Waters Co.), according to the manufacturer's recommended protocol. The purified product was dried with SAVANT SpeedVac Concentrator SPD2010 (Thermo Fisher Scientific), dissolved in 200 µL of a 50% acetonitrile aqueous solution and analyzed by high-performance liquid chromatography (LC-2030C, product of Shimadzu Corp.). Detection was at 280 nm using a Shimadzu PDA SPD-M20A as the detector. The column used was a Shim-Pack FC-ODS 150 mm * 4.6 mm (Shimadzu GLC). Solution A (0.1% formic acid aqueous solution) and solution B (100% methanol containing 0.1% formic acid) were used for elution. The elution was with a 12-minute linear concentration gradient from a 9:1 mixture to a 4:6 mixture of both, holding for 8 minutes, a 1-minute linear concentration gradient from a 4:6 mixture to a 0:10 mixture of both, holding for 1 minute, and continuing with the 0:10 mixture. The flow rate was 1.0 mL/min. As a control, the same experiment was carried out using transformed yeast with a transferred pESC-TRP vector lacking the insert. As a result, the reaction compound was detected in the culture solutions purified from the yeasts expressing D_DN4746 and Dca16994.1. In comparison with the sample, this compound was confirmed to be 2-hydroxynaringenin, being hydroxylated at the 2-position of the naringenin as the substrate (Fig. 4).

These results clearly demonstrated that D_DN4746 and Dca16994.1 exhibit specific hydroxylating activity at the 2-position of flavanones, and that D_DN4746 and Dca16994.1 are genes coding for proteins having hydroxylating activity at the 2-position of flavanones. Based on these results, these genes were identified as genes coding for proteins that hydroxylate the 2-position of flavanones.

[Sequence Listing]

## Claims

1. A polynucleotide encoding an enzyme with hydroxylating activity at the 2-position of flavanones, wherein the polynucleotide is selected from the group consisting of the following (A) to (E):
(A) polynucleotides consisting of the nucleotide sequences of SEQ ID NO: 1, 3, 5, 7, 9 or 11;
(B) polynucleotides that hybridize with polynucleotides consisting of nucleotide sequences complementary to the nucleotide sequences of SEQ ID NO: 1, 3, 5, 7, 9 or 11 under stringent conditions;
(C) polynucleotides encoding proteins consisting of the amino acid sequences of SEQ ID NO: 2, 4, 6, 8, 10 or 12;
(D) polynucleotides encoding proteins consisting of the amino acid sequences of SEQ ID NO: 2, 4, 6, 8, 10 or 12 having a deletion, substitution, insertion and/or addition of one or more amino acids; and
(E) polynucleotides encoding proteins having amino acid sequences with at least 90% identity to the amino acid sequences of SEQ ID NO: 2, 4, 6, 8, 10 or 12.

2. A polynucleotide according to claim 1, which is a polynucleotide consisting of the nucleotide sequence of SEQ ID NO: 1, 3, 5, 7, 9 or 11.

3. A polynucleotide according to claim 1, which is a polynucleotide encoding a protein consisting of the amino acid sequence of SEQ ID NO: 2, 4, 6, 8, 10 or 12.

4. A protein encoded by a polynucleotide according to any one of claims 1 to 3.

5. A vector that comprises a polynucleotide according to any one of claims 1 to 3.

6. A transgenic plant that comprises a polynucleotide according to any one of claims 1 to 3, or an inbred or outbred progeny of the same.

7. Propagules, partial plant bodies, tissue or cells of a transgenic plant according to claim 6, or its inbred or outbred progeny.

8. Cut flowers of a transgenic plant according to claim 6, or its inbred or outbred progeny, or a processed form created from the cut flowers.

9. A method for creating a transgenic plant, the method comprising a step of introducing into plant cells a polynucleotide encoding an enzyme that belongs to the 2-oxoglutaric acid-dependent dioxygenase (ODD) family and that has hydroxylating activity at the 2-position of flavanones.

10. The method according to claim 9, wherein the polynucleotide is selected from the group consisting of the following (A) to (E):
(A) polynucleotides consisting of the nucleotide sequences of SEQ ID NO: 1, 3, 5, 7, 9 or 11;
(B) polynucleotides that hybridize with polynucleotides consisting of nucleotide sequences complementary to the nucleotide sequences of SEQ ID NO: 1, 3, 5, 7, 9 or 11 under stringent conditions;
(C) polynucleotides encoding proteins consisting of the amino acid sequences of SEQ ID NO: 2, 4, 6, 8, 10 or 12;
(D) polynucleotides encoding proteins consisting of the amino acid sequences of SEQ ID NO: 2, 4, 6, 8, 10 or 12 having a deletion, substitution, insertion and/or addition of one or more amino acids; and
(E) polynucleotides encoding proteins having amino acid sequences with at least 90% identity to the amino acid sequences of SEQ ID NO: 2, 4, 6, 8, 10 or 12.

11. A non-human host that comprises a polynucleotide according to any one of claims 1 to 3.

12. A method for producing an enzyme with hydroxylating activity at the 2-position of flavanones, the method comprising:
a step of culturing a non-human host according to claim 11, and
a step of harvesting from the non-human host an enzyme with hydroxylating activity at the 2-position of flavanones.

13. A method for producing 2-hydroxyflavanone,
wherein the method includes contacting an enzyme produced by the method according to claim 12 with a flavanone.
